# EUROPEAN PATENT APPLICATION

(11) **EP 3 967 326 A1**
(43) Date of publication of application: **16.03.2022**
(21) Application number: 20398008.1
(22) Date of filing: 10.09.2020
(51) Int. Cl.: A61K 39/12, A61P 31/14

(54) **IMMUNOGENIC COMPOSITIONS**

(71) Applicant: Immunethep, SA, 3060-197 Cantanhede (PT)
(72) Inventor: The designation of the inventor has not yet been filed
(74) Representative: Cooley (UK) LLP

(57) **Abstract**

The invention provides immunogenic compositions and extends to their uses, for instance as a vaccine. The immunogenic compositions may be used for immunisation against severe acute respiratory syndrome coronavirus 2 (SARS-CoV-2), and may result in the prevention or reduction of infection by SARS-CoV-2.

## Description

### Field of the Invention

The present invention relates to diseases, disorders and conditions caused by severe acute respiratory syndrome coronavirus 2 (SARS-CoV-2), and particularly to coronavirus disease 2019 (COVID-19). The invention extends to immunogenic compositions and their uses, for instance as a vaccine. The immunogenic compositions may be used for immunisation against SARS-CoV-2, and may result in the prevention or reduction of infection by SARS-CoV-2. Immunisation may result in no or milder symptoms in subjects subsequently exposed to SARS-CoV-2.

### Background of the Invention

Historical clinical evidence demonstrates that vaccination is the best way to prevent and even eradicate infectious diseases. Classical strategies of producing vaccines take us back to Pasteur's days and the importance of *"identification, attenuation and inoculation"* of the pathogen. Although this classical approach was successful for most of the vaccines available nowadays, the development of new vaccines needs to take into account evolutionary mechanisms developed by many pathogens to escape host immunity. That may explain why vaccines for a number of pathogens have been difficult to develop.

Coronaviruses cause respiratory and intestinal infections in animals and humans. They were not considered to be highly pathogenic to humans until the outbreak of severe acute respiratory syndrome (SARS) in 2002. Ten years after SARS, another highly pathogenic coronavirus, Middle East respiratory syndrome coronavirus (MERS-CoV) emerged in Middle Eastern countries.

Starting around December 2019, an epidemic of pneumonia, which was named COVID-19 by the World Health Organization (WHO), broke out in Wuhan, China, and is spreading throughout the world. A new coronavirus, named SARS-CoV-2 by the Coronavirus Study Group of the International Committee on Taxonomy of Viruses was rapidly identified as the cause of disease (Wang, C., et al., J Med Virol, 2020; 92: 667-674). SARS-CoV-2 shares approximately 80% nucleotide sequence identity with SARS-CoV (the coronavirus responsible for SARS). However, the amino acid sequences of the seven conserved replicase domains in the open reading frame (ORF), ORF1ab, that were used for CoV species classification were 94.4% identical between SARS-CoV and SARS-CoV-2 (Zhou, P., et al., Nature, 2020; 579(7798): 270-273) .

To date, no SARS-CoV-2 vaccine has been approved for use in humans. Current research and development efforts involve an array of different technologies including viral vectors, nucleic acids, protein subunits and virus-like proteins. However, combating SARS-CoV-2 demands a vaccine that is both safe and potent because the transmission rates are high and the spread is difficult to track. This means many people need to be vaccinated in order to stop the spread and prevent deaths. Some of the aforementioned technologies have not been used in a licensed vaccine before and extensive safety tests will therefore be required before regulatory approval can be expected. There hence remains a need for development of a suitable SARS-CoV-2 vaccine.

### Summary of the Invention

In an aspect of the invention, there is provided an immunogenic composition comprising a plurality of whole SARS-CoV-2 viral particles.

The immunogenic compositions may be formulated for topical delivery to the respiratory tract. For instance, the immunogenic compositions may be formulated for intra-nasal administration or for oral inhalation.

The viral particles of the immunogenic compositions may be attenuated viral particles. The viral particles may be inactivated viral particles. The viral particles may be chemically inactivated viral particles, for instance β-propiolactone (β-PL)-inactivated viral particles or paraformaldehyde-inactivated viral particles.

The viral particles of the immunogenic compositions may be inactivated attenuated viral particles.

The immunogenic compositions may further comprise an adjuvant. For example, the adjuvant may be polyinosinic-polycytidylic acid (poly(I:C)) or a derivative thereof. In a particular example, the adjuvant may be poly(I:C) stabilised by lysine and carboxymethylcellulose (poly-ICLC).

The immunogenic compositions may be for use in a method of preventing or ameliorating disease or disease symptoms caused by or associated with SARS-CoV-2 infection in a subject.

The subject may be a human. The methods may comprise topically administering an immunogenic composition as disclosed herein to the respiratory tract of the subject. For instance, the methods may comprise intra-nasally administering or orally inhaling the immunogenic composition.

### Detailed Description

The data available in the literature indicate that strong sequence identity exists between all SARS-CoV-2 virus isolates. A sequence identity of 99.99% (99.91%-100%) was found at the nucleotide level and 99.99% (99.79%-100%) sequence identity at the amino acid level (Wang, C., *et al. supra*). However, although overall variation in ORF regions is low, 13 variation sites in 1a, 1b, S, 3a, M, 8, and N regions have been identified, among which positions nt28144 in ORF 8 and nt8782 in ORF 1a showed mutation rate of 30.53% (29/95) and 29.47% (28/95) respectively. These findings suggest that there may be selective mutations in SARS-CoV-2. The inventors have, therefore, deduced that new vaccines that target receptor binding domains, for example, may promote a rapid selective pressure for virus variants that escape vaccine recognition.

The inventors have noted, however, that the mutation rate within different isolates of the same virus is low if the whole structure is considered. Taking this into account, the inventors have deduced that a vaccine comprising whole virus would be the best strategy to prevent SARS-CoV-2 infections. By using .the whole virus, the inventors have sought to maximise the number of B-cell and T-cell epitopes - and thus, vaccine immunogenicity - and minimise the probability that one mutation could escape the vaccine.

The inventors also believe that another advantage of the use of whole viral antigens is that they provide longer antigen lifetimes than subunit vaccines, and thus continually re-stimulate T-cell responses to enhance both the breadth and duration of immunity.

Thus, in a first aspect of the invention, there is provided an immunogenic composition comprising a plurality of whole SARS-CoV-2 viral particles.

An "immunogenic composition", as used herein, is a composition that is suitable for inducing an immune response in a subject, wherein the immune response is at least in part directed to a specific antigen or specific antigens included in the immunogenic composition. In some embodiments, an immunogenic composition is for use as a vaccine. Suitably the composition is administered to a subject, for instance a human, in order to induce an immune response to an antigen or antigens included in the composition, such that the subject develops immunological memory to said antigen or antigens.

A "whole" viral particle is a viral particle that includes substantially all of the components of live virus. For SARS-CoV-2, such components typically include all of the virion structural molecules and all of the internal molecules. The virion structural molecules may include lipids, glycans, envelope (E) protein, spike (S) protein, and membrane (M) protein. The internal molecules may include RNA and the nucleocapsid (N) protein. In some embodiments, such particles are produced by means of a cell line. However, any means of production may be used to generate whole viral particles. Viral particles that include any alterations which may result from the processes of attenuation, production, purification, inactivation, and/or formulation as a vaccine are encompassed by the term "whole" viral particle.

A "SARS-CoV-2" viral particle may be of any SARS-CoV-2 viral strain. For instance, any SARS-CoV-2 viral strain associated with the COVID-19 pandemic. In a particular example, the SARS-CoV-2 viral strain has the genome according to NCBI Reference Sequence: NC_045512.2 (https://www.ncbi.nlm.nih.gov/nuccore/NC-045512.2). Also encompassed are SARS-CoV-2 viral strains that have been modified or altered, but are directly or indirectly derived from any SARS-CoV-2 viral strain. SARS-CoV-2 viral strains that may occur naturally after the COVID-19 pandemic has been declared to be at an end are explicitly encompassed. In particular, any SARS-CoV-2 viral strain that becomes established as a circulating strain in humans or animals is encompassed by this term, as are modified or altered strains that are directly or indirectly derived from said circulating strain.

In some embodiments, the SARS-CoV-2 viral particles are derived from a SARS-CoV-2 viral strain with a genome that has at least about 70%, at least about 80%, at least about 90%, at least about 95%, at least about 99%, at least about 99.5%, at least about 99.9%, at least about 99.95%, at least about 99.96%, at least about 99.97%, at least about 99.98%, at least about 99.99%, or 100% sequence identity with NCBI Reference Sequence: NC_045512.2.

Due to the degeneracy of the genetic code, protein-coding sequences within the SARS-CoV-2 genome can be varied or changed without affecting the sequence of the encoded polypeptide or protein. For instance, the protein-coding sequences could be altered by the substitution of different codons that encode the same amino acid within the sequence, thus producing a silent change. Thus, in some embodiments, the SARS-CoV-2 viral particles are derived from a SARS-CoV-2 viral strain with a genome that has at least 70%, at least about 80%, at least about 90%, at least about 95%, at least about 99%, at least about 99.5%, at least about 99.9%, at least about 99.95%, at least about 99.96%, at least about 99.97%, at least about 99.98%, at least about 99.99%, or 100% sequence identity with NCBI Reference Sequence: NC_045512.2, and wherein silent changes in a protein coding sequence are not considered when calculating identity.

In some embodiments, the SARS-CoV-2 viral particles are derived from a SARS-CoV-2 viral strain with a genome for which the open reading frames have at least about 70%, at least about 80%, at least about 90%, at least about 95%, at least about 99%, at least about 99.5%, at least about 99.9%, at least about 99.95%, at least about 99.96%, at least about 99.97%, at least about 99.98%, at least about 99.99%, or 100% sequence identity with the open reading frames of NCBI Reference Sequence: NC_045512.2. In particular, the 13 identified variation sites in the 1a, 1b, S, 3a, M, 8, and N regions (Wang, C., *et al. supra*), including positions nt28144 in ORF 8 and nt8782 in ORF 1a, may vary from NCBI Reference Sequence: NC_045512.2.

In other embodiments, the SARS-CoV-2 viral particles comprise all SARS-CoV-2 structural proteins (*e.g*. the E protein, S protein, M protein, N protein, and accessory proteins found in the virion) wherein any one or all of the structural proteins have at least about 70%, at least about 80%, at least about 90%, at least about 95%, at least about 96%, at least about 97%, at least about 98%, at least about 99%, at least about 99.5%, at least about 99.9%, or 100% sequence identity with those encoded in NCBI Reference Sequence: NC_045512.2.

In other embodiments, the SARS-CoV-2 viral particles comprise an E protein, an S protein, an M protein, and an N protein wherein any one or all of the proteins have at least about 70%, at least about 80%, at least about 90%, at least about 95%, at least about 96%, at least about 97%, at least about 98%, at least about 99%, at least about 99.5%, at least about 99.9%, or 100% sequence identity with those encoded in NCBI Reference Sequence: NC_045512.2.

In yet further embodiments, the SARS-CoV-2 viral particles comprise an E protein, an S protein, an M protein, and an N protein as encoded in NCBI Reference Sequence: NC_045512.2, further comprising one, two, three, four, five, six, seven, eight, nine, or 10 amino acid residue substitutions in any one of the proteins, or one, two, three, four, five, six, seven, eight, nine, or 10 amino acid residue substitutions across all of said proteins. In some embodiments, the proteins may each comprise from one to 10, from one to five, or from one to three substitutions. In some embodiments, the substitutions are conservative substitutions.

A conservative substitution is the replacement of an amino acid residue with another amino acid residue with a side chain of similar biophysical properties. For example, small non-polar, hydrophobic amino acids include glycine, alanine, leucine, isoleucine, valine, proline, and methionine. Large non-polar, hydrophobic amino acids include phenylalanine, tryptophan, and tyrosine. The polar neutral amino acids include serine, threonine, cysteine, asparagine, and glutamine. The positively charged (basic) amino acids include lysine, arginine, and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

The skilled technician will appreciate how to calculate the percentage identity between two amino acid/nucleotide/peptide sequences. In order to calculate the percentage identity between two amino acid/nucleotide/peptide sequences, an alignment of the two sequences must first be prepared, followed by calculation of the sequence identity value. The percentage identity for two sequences may take different values depending on: (i) the method used to align the sequences, for example, ClustalW, BLAST, FASTA, Smith-Waterman (implemented in different programs), or structural alignment from 3D comparison; and (ii) the parameters used by the alignment method, for example, local *versus* global alignment, the pair-score matrix used (for example, BLOSUM62, PAM250, Gonnet etc.) and gap-penalty, for example, functional form and constants.

Having made the alignment, there are many different ways of calculating percentage identity between the two sequences.

For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length-dependent. Therefore, the shorter a pair of sequences is, the higher the sequence identity one may expect to occur by chance.

Hence, it will be appreciated that the accurate alignment of amino acid or nucleic acid sequences is a complex process. The popular multiple alignment program ClustalW is a method for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/nucleotide/peptide sequences may then be calculated from such an alignment as (N/T)^{∗}100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula: Sequence Identity = (N/T)^{∗}100.

The sequence alignment may be a pairwise sequence alignment. Suitable services include Needle (EMBOSS), Stretcher (EMBOSS), Water (EMBOSS), Matcher (EMBOSS), LALIGN, or GeneWise. In an example, the identity between two protein sequences may be calculated using the service Matcher (EMBOSS) set to the default parameters, *e.g.* matrix (BLOSUM62), gap open (14), gap extend (4), alternative matches (1). In an example, the identity between two DNA sequences may be calculated using the service Matcher (EMBOSS) set to the default parameters, *e.g.* matrix (DNAfull), gap open (16), gap extend (4), alternative matches (1).

The immunogenic compositions of the invention comprise a plurality of whole SARS-CoV-2 viral particles. A "plurality" can mean a therapeutically effective amount of whole SARS-CoV-2 viral particles. A "therapeutically effective amount" of an agent is any amount which, when administered to a subject, is the amount of agent that is needed to produce the desired effect (for instance, the prevention or amelioration of disease or disease symptoms caused by or associated with SARS-CoV-2 infection).

In an embodiment, an immunogenic composition of the invention comprises a dose of about 0.1 to about 100 µg, about o.5 to about 50 µg, about 0.75 to about 25 µg, about 0.85 to about 15 µg, or from about 1 to about 10 µg of whole SARS-CoV-2 viral particles.

The inventors have also deduced that topical (local) delivery of the immunogenic compositions to the respiratory tract, *e.g.* via intra-nasal administration, may be important to maximise lung immunity. FluMist, a nasally administered influenza vaccine, induces much greater lung-localised viral strain-specific T-cell responses than the typical injected flu vaccine (the latter, however, preferentially induces strain-specific neutralising antibodies). Without being bound to a particular theory, the inventors have hypothesised that a tissue-specific immune response, and hence residual tissue-specific immune memory, may be an important feature of a SARS-CoV-2 vaccine due to the promotion of lung immunity and the avoidance of viral entry into lung cells.

Therefore, in some embodiments, the immunogenic compositions are formulated for topical delivery to the respiratory tract. In particular embodiments, the immunogenic compositions are formulated for intra-nasal administration. In other embodiments, the immunogenic compositions are formulated for oral inhalation. The immunogenic compositions may be formulated for administration to human subjects.

In further support of the approach disclosed herein, the inventors have recognised that multivalent rotavirus vaccines also decrease coronavirus-induced acute gastroenteritis. Rotavirus vaccines are live attenuated orally administered vaccines that promote strong immunity to multiple viral epitopes in the mucosa and bronchus-associated lymphoid tissue. Although these data are far from conclusive, the inventors believe that local immunisation applied to the target tissues can favour specific viral responses in those tissues and, again, provide reason for the use of a whole virus vaccine delivered in a tissue-specific manner.

The immunogenic compositions may comprise a pharmaceutically acceptable vehicle for increasing the suitability of the composition for topical delivery to the respiratory tract, *e.g*. via intra-nasal administration or oral inhalation. A "pharmaceutically acceptable vehicle", as referred to herein, is any known compound or combination of known compounds that are known to those skilled in the art to be useful in formulating pharmaceutical compositions.

The immunogenic compositions may comprise a pharmaceutically acceptable buffer or buffers. The buffer or buffers may maintain the pH of the immunogenic compositions at a level suitable for topical delivery to the respiratory tract, *e.g.* via intra-nasal administration or oral inhalation.

The immunogenic compositions may be in the form of a powder, aerosol, liquid, solid insert, or nasal gel.

The immunogenic compositions may include one or more substances which may also act as flavouring agents, lubricants, solubilisers, suspending agents, dyes, fillers, glidants, inert binders, sweeteners, preservatives, or dyes.

The pharmaceutically acceptable vehicle may also be an encapsulating material. In powders, the vehicle may be a finely divided solid that is in admixture with the finely divided active agents according to the invention. In another embodiment, the pharmaceutically acceptable vehicle is a gel.

The pharmaceutically acceptable vehicle may be a liquid and the immunogenic compositions may be in the form of a solution. The liquid vehicle can contain other suitable pharmaceutical additives such as solubilisers, emulsifiers, buffers, preservatives, sweeteners, flavouring agents, suspending agents, thickening agents, colours, viscosity regulators, stabilisers or osmo-regulators.

Formulations for nasal vaccine delivery are known and commercially available (Yusuf, H., et al., Hum Vacc Immunother, 2017; 13(1): 34-45). Any of these formulations may be employed in the present invention. Formulations for oral inhalation of a vaccine are also known; these may be administered in the form of a fine powder or aerosol, for example, using a Diskhaler^{®} or Turbohaler^{®}.

As discussed above, the inventors make use of whole viral particles. Without treatment, such particles could be infectious. Therefore, to increase the suitability of the immunogenic compositions for mass production and use, in some embodiments the viral particles are attenuated whole SARS-CoV-2 viral particles.

The principle of "attenuated" viral particles is known to those skilled in the art. In summary, attenuated viral particles are less virulent than non-attenuated viral particles. Viral strains may be attenuated by passaging said viral strains through a cell line, hence adapting the strain to production in said cells. In an embodiment, the viral particles have been attenuated by serial passages in at least one cell line. In an embodiment, the viral particles have been attenuated by at least 1, 2, 3, 4, 5, 6, 7, 8, 9, 10, 20, 50, 0r 100 passages in a cell line. The cell line may be any cell line suitable for this purpose, including cell lines modified to express, or endogenously expressing, the angiotensin converting enzyme II (ACE2) receptor or human ACE2 receptor. In a particular embodiment, the at least one cell line is Vero cells.

As discussed above, immunogenic compositions comprising whole viral particles could be infectious. Therefore, to increase the suitability of the immunogenic compositions for mass use, in some embodiments the viral particles are inactivated whole SARS-CoV-2 viral particles. In some embodiments, the viral particles are inactivated attenuated whole SARS-CoV-2 viral particles.

An "inactivated" viral particle is a viral particle that has been treated such that it cannot cause disease in a host. The inactivated viral particle may be unable to infect a host cell in a manner that would lead to viral replication. The inactivated viral particle may have been "killed" to prevent infectivity. Methods of inactivating viral particles are known in the art, and may involve heat or chemical inactivation. In a particular embodiment, the viral particle of the invention is chemically inactivated.

The chemical inactivation may be through the use of β-PL. In some embodiments, the inactivation is through the use of β-PL at a concentration of about 0.001% to about 10%, about 0.01% to about 1%, about 0.05% to about 0.5%, or, in a particular embodiment, about 0.1% (w/v). The viral particles may be exposed to the β-PL for at least about one hour, at least about six hours, at least about 16 hours, at least about 24 hours, at least about 48 hours, or at least about 72 hours. In a particular embodiment, the viral particles are exposed to β-PL for about 48 hours. During exposure to β-PL, the viral particles may be maintained at approximately 3 °C-7 °C, or, in a particular embodiment, about 4 °C. In an embodiment, the viral particles are exposed to about 0.1% (w/v) β-PL for about 48 hours at about 4 °C.

The chemical inactivation may be through the use of paraformaldehyde (also known as formalin). In some embodiments, the inactivation is through the use of paraformaldehyde at a concentration of about 0.002% to about 20%, about 0.02% to about 2%, about 0.05% to about 0.5%, or, in a particular embodiment, about 0.2% (w/v). The viral particles may be exposed to the paraformaldehyde for at least about one hour, at least about six hours, at least about 16 hours, at least about 24 hours, at least about 48 hours, or at least about 72 hours. The viral particles may be exposed to the paraformaldehyde for at least about four days, at least about five days, at least about six days, or at least about seven days. In a particular embodiment, the viral particles are exposed to the paraformaldehyde for about five days. During exposure to paraformaldehyde, the viral particles may be maintained at approximately 25 °C-37 °C, 30 °C-35 °C or, in a particular embodiment, about 32 °C. In an embodiment, the viral particles are exposed to about 0.2% (w/v) paraformaldehyde for about five days at about 32 °C.

To increase the immunogenicity of the immunogenic compositions, an adjuvant may be included. The adjuvant may be any that is licensed for human use. Examples of adjuvants include: cholera toxin, a squalene-like molecule, aluminium hydroxide (alum), tetanus toxoid, or diphtheria toxin. The adjuvant may be a Toll-like Receptor (TLR)-3 agonist. The adjuvant may be an analogue, mimic, or derivative of double stranded RNA. Other possible adjuvants include oil-in-water emulsions, such as MF59 and AS03.

Poly(I:C) is an adjuvant that can elicit appropriate innate responses to enhance viral immunity. The inventors have noted that a poly(I:C) analogue has already been tested as a therapeutic agent for SARS in mice, and poly(I:C) stabilised by lysine and carboxymethylcellulose (poly-ICLC) showed a very nice tolerability profile and good adjuvant effect when used in human clinical trials (Okada, H., et al., J Clin Oncol, 2011; 29(3): 330-336). In addition, the inventors have deduced that poly(I:C) is a suitable adjuvant for use with the present invention due to its ability to i) promote humoral immunity, ii) increase virus-specific CD8+ T-cell activation by increasing cross-presentation of viral-associated antigens, and iii) to enhance innate immunity to virus. Thus, in an embodiment, the adjuvant is poly(I:C) or a derivative thereof. In a particular embodiment, the adjuvant is poly-ICLC.

In a particular embodiment, there is provided an immunogenic composition comprising a plurality of whole SARS-CoV-2 viral particles and an adjuvant, wherein the immunogenic composition is formulated for intra-nasal administration.

In another particular embodiment, there is provided an immunogenic composition comprising a plurality of whole SARS-CoV-2 viral particles and an adjuvant, wherein the immunogenic composition is formulated for oral inhalation.

In another particular embodiment, there is provided an immunogenic composition comprising a plurality of inactivated attenuated whole SARS-CoV-2 viral particles and an adjuvant.

In a yet another embodiment, there is provided an immunogenic composition comprising a plurality of inactivated attenuated whole SARS-CoV-2 viral particles and poly(I:C) or a derivative thereof, such as poly-ICLC, wherein the immunogenic composition is formulated for intra-nasal administration or oral inhalation.

In a second aspect of the invention, there is provided an immunogenic composition of the first aspect of the invention, for use in therapy.

In a third aspect, there is provided an immunogenic composition of the first or second aspect for use in a method of preventing or ameliorating disease or disease symptoms caused by or associated with SARS-CoV-2 infection in a subject.

In an embodiment, the method comprises administering the immunogenic composition to the subject in order to elicit immunological memory to an antigen or antigens associated with the SARS-CoV-2 viral particles. The immunological memory may prevent later SARS-CoV-2 infection, or may reduce the severity of symptoms associated with later SARS-CoV-2 infection.

In an embodiment, the subject is an animal subject, for instance a jawed-vertebrate, mammal, or domestic animal. Hence, the immunogenic compositions according to the invention may be used to treat any mammal, for example livestock (for example, a horse), pets, or may be used in other veterinary applications. In a particular embodiment, the subject is a human.

In an embodiment, the method comprises topically delivering the immunogenic composition to the respiratory tract of the subject. For instance, in some embodiments, the method comprises administering the immunogenic composition intra-nasally. The immunogenic composition may be administered in the form of a powder, aerosol, liquid, solid insert, or nasal gel. The method may thus comprise administering the immunogenic composition by insufflation, nasal inhalation or mucosally (e.g. by nasal drops, gels or inserts). Intra-nasal administration may suitably be in the form of a fine powder or aerosol nasal spray using a modified Diskhaler^{®} or Turbohaler^{®}. In other embodiments, the method comprises administering the immunogenic composition by oral inhalation. The immunogenic composition may be administered in the form of a powder, aerosol or liquid. The vaccine is suitably administered in the form of a fine powder or aerosol via a Diskhaler^{®} or Turbohaler^{®}.

A suitable dosing regimen may be used depending on the organism to be vaccinated. In some embodiments, the method comprises administering a therapeutically effective amount of the immunogenic composition. In some embodiments, the method comprises administering a dose of the immunogenic composition to the subject of about 0.1 to about 100 µg, about o.5 to about 50 µg, about 0.75 to about 25 µg, about 0.85 to about 15 µg, or from about 1 to about 10 µg. In some embodiments, the method comprises administering the immunogenic composition once to the subject. In other embodiments, the subject receives multiple doses of the immunogenic composition. The amount of immunogenic composition to be administered may be the amount sufficient to induce protective levels of anti-SARS-CoV-2 antibodies or neutralising anti-SARS-CoV-2 antibodies in a subject. Blood may be withdrawn for analysis of serum (IgG) responses. Saliva, vaginal fluids or faeces may be taken for analysis of mucosal (secretory IgA) responses. Indirect enzyme-linked immunosorbent assay (ELISA) may be used to analyse antibody responses in serum and mucosal samples, to gauge the efficacy of the administration of the immunogenic compositions.

In some embodiments, the method comprises measuring the levels of anti-SARS-CoV-2 antibodies or neutralising anti-SARS-CoV-2 antibodies in the subject, and administering further doses of the immunogenic composition should levels of said antibodies fail to reach a protective threshold, or should said levels fall below a protective threshold. A protective threshold may be the threshold at which exposure to SARS-CoV-2 does not result in infection, the threshold at which exposure to SARS-CoV-2 does not result in symptomatic infection, or the threshold at which exposure to SARS-CoV-2 results in less severe infection in comparison to untreated subjects.
In some embodiments, the subject has not been infected with SARS-CoV-2. In other embodiments, the subject currently has or previously had a mild SARS-CoV-2 infection. In other embodiments, the subject has no or low levels of anti-SARS-CoV-2 antibodies, or has no or low levels of neutralising anti-SARS-CoV-2 antibodies.

It will be appreciated that the immunogenic compositions disclosed herein may be used in a monotherapy. Alternatively, the immunogenic compositions disclosed herein may be used as an adjunct to, or in combination with, known therapies for treating, ameliorating, or preventing infections with SARS-CoV-2.

In a particular embodiment of the invention, there is provided a method of preventing or ameliorating disease or disease symptoms caused by or associated with SARS-CoV-2 infection in a subject, comprising topically administering a therapeutically effective amount of an immunogenic composition of the first aspect of the invention to the respiratory tract of said subject. In an advantageous embodiment, the immunogenic compositions are delivered intra-nasally or via oral inhalation.

All of the features described herein (including any accompanying claims, abstract and drawings), and/or all of the steps of any method or process so disclosed, may be combined with any of the above aspects in any combination, except combinations where at least some of such features and/or steps are mutually exclusive. Methods and materials similar to or equivalent to those described herein can be used in the practice and testing of the invention, and suitable methods and materials are described below.

### Examples

### Example 1: Production of SARS-CoV-2 vaccine

A clinical isolate of SARS-CoV-2 is taken from an infected patient with mild symptoms of COVID-19 (obtained from INSA - Instituto Nacional de Saúde Ricardo Jorge, Portugal) and used to infect Vero cells, as described elsewhere (Ng, M.L., et al., J Gen Virol, 2003; 84(12): 3291-3303 and Keyaerts, E., et al., Biochem Biophys Res Commun, 2005; 329(3): 1147-1151). The virus is replicated until reaching a titre of approximately 10⁷ pfu/ml, which is used to infect new cell monolayers.

Attenuation of the virus is performed by serial passages of the virus in Vero cells as previously described for other vaccines (Bernstein, D.I., Semin Pediatr Infect Dis, 2006; 17(4):188-194).

For chemical inactivation of the virus, paraformaldehyde (formalin) or β-PL is used. Virus-infected culture supernatant is inactivated using either β-PL at a concentration of 0.1% (w/v) and kept for 48 hours at 4 °C or formalin at a concentration of 0.2% (w/v) and kept for 5 days at 32 °C.

Vaccine formulations for intra-nasal administration are prepared in saline solution using poly-ICLC as adjuvant. Each vaccine formulation contains live-attenuated SARS-CoV-2, β-PL-inactivated SARS-CoV-2 or formalin-inactivated SARS-CoV-2.

### Example 2: Immunogenicity of SARS-CoV-2 vaccine

The immunogenicity of the vaccine formulations prepared in Example 1 is tested by evaluating and comparing IgG titres obtained after intra-nasal (i.n.) immunisation in adult BALB/c mice.

Mice receive two doses of the intra-nasal SARS-CoV-2 vaccine, with a three-week interval between doses. Controls are sham-immunised (i.n.) with the vaccine vehicle plus 0.5 µg of poly-ICLC as adjuvant.

Sera are collected from the mice one month after the last dose and specific virus IgG titres are evaluated by ELISA.

### Example 3: In vitro potency assay

The ability of the SARS-CoV-2 vaccine formulations produced in Example 1 to stop virus replication is assessed using an *in vitro* potency assay.

Total. IgG is purified from sera of mice immunised with inactivated virus or live-attenuated virus as per Example 2, using protein-G columns (GE Healthcare). Different concentrations of IgG from immunised mice are added to SARS-CoV-2-infected Vero cells and the ability of this IgG to stop virus replication is evaluated. Human IgG purified from the peripheral blood of individuals that recovered from COVID-19 is also tested as a control.

### Example 4: In vivo efficacy trials using mice as an animal model

For the *in vivo* efficacy trials BALB/c mice are used as an animal model. BALB/c mice express ACE2 on lung cells. Although the spike protein of SARS-CoVs has a much higher binding affinity to human ACE2 than to those of mice, SARS-CoVs also bind mouse ACE2 and cause lung pathology in mice. BALB/c mice are immunised i.n. as described in Example 2 with live-attenuated virus or inactivated virus plus 0.5 µg poly-ICLC as adjuvant.

Two different control groups are used: one control group is sham-immunised (i.n.) with saline solution alone (vehicle) and the other control group is sham-immunised (i.n.) with 0.5 µg of poly-ICLC. This allows the therapeutic effect of poly-ICLC alone to be evaluated.

Two weeks after the last immunisation, the mice are challenged i.n. (20 µl in the left nostril) with 4 x 10⁶ pfu of the SARS-CoV-2 (obtained from INSA - Instituto Nacional de Saúde Ricardo Jorge, Portugal) corresponding to an 80% tissue culture infective dose (TCID80)).

Body weight is measured daily for 20 days. Infected animals are also sacrificed at various time points after inoculation to analyse virus replication, haematology, cytokine expression, and pathology as a measure of *in vivo* efficacy.

### Example 5: Toxicity trials in the rat model

The purpose of this study is to assess any possible toxic effects of the SARS-CoV-2 vaccine in rats after repeated i.n. administration over a period of four weeks.

60 rats are assigned to three groups each containing 10 males and 10 females, including one test item vaccine group (vaccine group), one adjuvant control group and one vehicle control group. An additional 30 rats are assigned to three groups (vaccine, adjuvant control and vehicle control groups) each containing five males and five females, to serve as recovery animals which are observed for a period of four weeks following the last administration.

The test item vaccine, adjuvant and vehicle are administered once weekly at a single i.n. administration (in total five administrations), with the application volume of 20 µl per animal (left nostril).

All animals are necropsied and examined *post-mortem,* and histological examinations performed on different organs and tissues. Necropsies and tissue-collection for histopathology are performed. Briefly, one day after the last administration or at the end of the recovery period, animals are deeply anesthetised using ketamine/xylazin. After blood sampling from the abdominal aorta, a detailed gross necropsy which includes careful examination of the external surface of the body, all orifices and the cranial, thoracic and abdominal cavities and their content is performed.

After necropsy, organs and tissues are collected, and then fixed with and preserved in 4% (w/v) neutral-buffered formaldehyde except eyes, testes and epididymides that are fixed in modified Davidson's fixative for approximately 24 hours before preserving them in 70% (w/v) ethanol. Histotechnique is performed on collected and preserved tissue samples.

In order to allow a detection of possible delayed occurrence or persistence of or recovery from toxic effects, the animals in the recovery groups are observed for a period of four weeks following the last administration. As part of these assessments, histopathological examination is performed.

## Claims

1. An immunogenic composition comprising a plurality of whole SARS-CoV-2 viral particles.

2. An immunogenic composition according to claim 1, wherein the immunogenic composition is formulated for topical delivery to the respiratory tract.

3. An immunogenic composition according to claim 2, wherein the immunogenic composition is formulated for intra-nasal administration or for oral inhalation.

4. An immunogenic composition according to any preceding claim, wherein the viral particles are attenuated viral particles.

5. An immunogenic composition according to any preceding claim, wherein the viral particles are inactivated viral particles.

6. An immunogenic composition according to claim 5, wherein the inactivated viral particles are chemically inactivated viral particles.

7. An immunogenic composition according to claim 6, wherein the chemically inactivated viral particles are:
(a) β-propiolactone (β-PL)-inactivated viral particles; or
(b) paraformaldehyde-inactivated viral particles.

8. An immunogenic composition according to any preceding claim, wherein the viral particles are inactivated attenuated viral particles.

9. An immunogenic composition according to any preceding claim, further comprising an adjuvant.

10. An immunogenic composition according to claim 9, wherein the adjuvant is polyinosinic-polycytidylic acid (poly(I:C)) or a derivative thereof.

11. An immunogenic composition according to claim 10, wherein the adjuvant is poly(I:C) stabilised by lysine and carboxymethylcellulose (poly-ICLC).

12. An immunogenic composition according to any preceding claim, for use in a method of preventing or ameliorating disease or disease symptoms caused by or associated with SARS-CoV-2 infection in a subject.

13. An immunogenic composition for use according to claim 12, wherein the subject is a human.

14. An immunogenic composition for use according to claim 12 or 13, wherein the method comprises topically administering the immunogenic composition to the respiratory tract of the subject.

15. An immunogenic composition for use according to any one of claims 12 to 14, wherein the method comprises intra-nasally administering or orally inhaling the immunogenic composition.
